# EUROPÄISCHE PATENTSCHRIFT

(11) **EP 0 505 742 B1**
(45) Veröffentlichungstag und Bekanntmachung des Hinweises auf die Patenterteilung: **20.09.2000**
(21) Anmeldenummer: 92103113.4
(22) Anmeldetag: 25.02.1992
(51) Int. Cl.: C07D 207/34, C07D 317/46, C07D 405/04, C07C 22/08, C07C 22/04, C07C 255/34

(54) **Verfahren zur Herstellung von in 3-Stellung substituierten 4-Cyano-pyrrolverbindungen**
Process for the production of 4-cyanopyrrole compounds substituted at the 3-position
Procédé pour la préparation de 4-cyanopyrroles substitués en position 3-

(30) Priorität: 08.03.1991 DE 4107398
(43) Veröffentlichungstag der Anmeldung: 30.09.1992
(73) Patentinhaber: BAYER AG, 51368 Leverkusen (DE)
(72) Erfinder: Knüppel, Peter C., Dr., W-5632 Wermelsksirchen 3 (DE); Lantzsch, Reinhard, Dr., W-5600 Wuppertal (DE); Jelich, Klaus, Dr., W-5600 Wuppertal (DE); Andres, Peter, Dr., W - 5090 Leverkusen 3 (DE); Marhold, Albrecht, Dr., W-5090 Leverkusen (DE)

(56) Entgegenhaltungen:
- EP-A- 0 206 999
- EP-A- 0 310 558
- EP-A- 0 318 704
- EP-A- 0 378 046
- DE-A- 2 927 480
- DE-A- 3 800 387

## Beschreibung

Die Erfindung betrifft ein neues Verfahren zur Herstellung von in 3-Stellung substituierten 4-Cyano-pyrrolverbindungen, welche als Schädlingsbekämpfungsmittel verwendet werden können.

Es ist bekannt, daß fungizid wirksame 3-Aryl-4-cyanopyrrolverbindungen erhältlich sind, wenn man entsprechend substituierte Zimtsäurenitrile mit p-Toluolsulfonylmethylisocyanid (TOSMIC) in Gegenwart von Natriumhydrid umsetzt (vergleiche DE-OS 29 27 480 oder Tetrahedron Lett. 52, 5337 [1972]). Dieses Verfahren bringt jedoch mit ca. 35-45 % Ausbeute nur unbefriedigende Ergebnisse. Nachteilig ist außerdem, daß die so erhältlichen Verbindungen aufwendig gereinigt werden müssen (vergleiche JP 61 30 571). Schließlich sind die Reagenzien Natriumhydrid und p-Toluolsulfonylmethylisocyanid (TOSMIC) beide für technische Synthesen nicht sehr gut geeignet, ersteres wegen der hohen Hydrolyseanfälligkeit und der damit verbundenen Brandgefahr des bei der Hydrolyse freigesetzten gasförmigen Wasserstoffes, das zweite wegen der leichten Zersetzlichkeit bei erhöhter Temperatur (vergleiche EP 174 910).

Nicht besser geeignet für technische Synthesen ist das in einem analogen Verfahren als Base verwendete n-Butyllithium (vergleiche EP 333 661), welches ebenfalls sehr hydrolyseanfällig und darüberhinaus aufwendig und teuer herzustellen ist. Die Benutzung anderer Basen wird beschrieben, aber auch dabei werden nur unbefriedigende Umsätze erzielt (vergleiche z. B. EP 310 558 oder EP 206 999).

Weiterhin ist bekannt, daß man die für die Synthese von 3-Aryl-4-cyano-pyrrolverbindungen erforderlichen Zimtsäurenitrile durch literaturbekannte Verfahren, wie beispielsweise durch WITTIG-Reaktion (vergleiche z. B. Tetrahedron 41, 1259 [1985] oder J. Amer. Chem. Soc. 83, 1733 [1961] oder Synthesis 1977, 126) oder durch KNOEVENAGEL-Kondensation (vergleiche z. B. Tetrahedron 43, 537 [1987] oder Tetrahedron Lett. 1979, 553 oder EP 378 046) aus Benzaldehyden erhält. Die Synthese der hierfür erforderlichen Benzaldehyde ist jedoch außerordentlich schwierig, umständlich und verläuft über viele Stufen (vergleiche z. B. EP 61 907 oder JP 60 38 336 oder EP 333 658).

Auch die großtechnische Herstellung der als fungizid wirksamen Endprodukte erwünschten in 3-Stellung substituieten 4-Cyano-pyrrolverbindungen über den literaturbekannten Weg der α-substituierten Zimtsäurenitrile (vergleiche z. B. DE-OS 37 18 375 oder DE- OS 38 00 387 oder EP 324 336 oder EP 378 046) scheitert letztlich daran, daß hierzu als Ausgangsverbindungen zunächst diese sehr schwer zugänglichen Benzaldehyde erforderlich sind.

Die alternative Herstellung der als Vorprodukte erforderlichen Zimtsäurenitrile aus entsprechenden aromatischen Aminen mittels MEERWEIN-Arylierung liefert gerade im Falle der erwünschten 2,3-disubstituierten Aryl-Verbindung sehr schlechte Ausbeuten (vergleiche EP 318 704 oder EP 206 999 oder J. Org. Chem. 34, 714 [1969]).

Schließlich ist bekannt, daß man die als Vorprodukte erforderlichen Zimtsäurenitrile auch erhalten kann, wenn man Bromide oder Jodide mit Acrylnitril in Gegenwart eines Palladiumkatalysators und eines Phosphines als Reaktionshilfsmittel in basischen oder dipolar-aprotischen Lösungsmitteln wie beispielsweise Triethylamin oder Dimethylformamid umsetzt (vergleiche z. B. EP 78 768 oder US 4.820.835 oder J. Chem. Soc., Perkin Trans. 1, 2597 [1987] oder J. Organomet. Chem. 258, 101, [1983]). Auch dieses Verfahren versagt jedoch im Falle der 2,3-disubstituierten Arylverbindungen. Unter den literaturbekannten Reaktionsbedingungen wurde nur ein geringer Umsatz zu dem gewünschten Endprodukt erzielt (vergleiche das Herstellungsbeispiel III-1).

Es wurde nun gefunden, daß man die in 3-Stellung substituierten 4-Cyano-pyrrolverbindungen der Formel 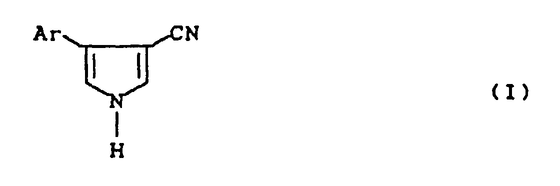 in welcher
- Ar: für 2,3-Dichlorphenyl, 2,3-Difluorphenyl, 2-Fluor-3-chlorphenyl, 2-Chlor-3-fluorphenyl, 2,4-Difluor-3-chlorphenyl, 2-Fluor-3-trifluormethylphenyl oder 2,2-Difluorbenzodioxolyl steht,
durch Umsetzung von Bromiden der Formel

Ar―Br (II)

in welcher
- Ar: die oben angegebene Bedeutung hat,
in erster Stufe mit Acrylnitril in Gegenwart eines Lösungsmittels und eines geeigneten Reaktionshilfsmittels zu den Acrylnitril-Derviaten der Formel

Ar―CH=CH―CN (III)

in welcher
- Ar: die oben angegebene Bedeutung hat,
und
in einer zweiten Stufe mit Phenylsulfonylmethylisocyaniden der Formel

Ph―SO₂―CH₂―NC (IV)

in welcher
- Ph: für 4-Methylphenyl oder 4-Chlorphenyl steht,
in Gegenwart eines geeigneten Verdünnungsmittels und gegebenenfalls in Gegenwart eines geeigneten Reaktionshilfsmittels,
nach einem Verfahren erhält, das dadurch gekennzeichnet ist,
daß man die erste Stufe in Gegenwart eines Palladiumkatalysators und die zweite Stufe in Gegenwart eines 1,2- bis 1,8fach molaren Überschusses an Alkalimetallalkoholat oder Alkalimetallhydroxid, bezogen auf Acrylnitril-Derivat, durchführt.

Überraschenderweise gelingt bei dem erfindungsgemäßen Verfahren die Umsetzung der Acrylnitril-Derivate der Formel (III) mit Phenylsulfonyl-methylisocyaniden der Formel (IV) in der zweiten Stufe in Gegenwart eines 1,2 bis 1,8fachen molaren Überschusses an Alkalimetallalkoholat oder Alkalimetallhydroxid als Base in sehr hohen Ausbeuten, obwohl aus dem Stand der Technik bekannt war, daß schwächere Basen als Natriumhydrid, also auch Alkalimetallalkoholate oder Alkalimetallhydroxide, nur schlechte Ausbeuten liefern (vergleiche z. B. EP 310 558 oder EP 206 999).

Gegen die Verwendung eines Basenüberschusses sprach außerdem noch die Annahme, daß ein solcher Überschuß an Base in der Reaktionsmischung mit den verwendeten Ausgangeprodukten ebenso wie mit dem Endprodukt reagieren könnte und daß diese Nebenreaktionen zu unerwünschten Nebenprodukten und einer deutlichen Verminderung der Ausbeute an erwünschtem Endprodukt führen würden.
(Zu Nebenreaktionen Base und Tosmic vergleiche z. B. Tetrahedron Lett. 1972, 2363-2368; Angew. Chem. 83, 357-358 [1971]; Angew. Chem. 86, 878ff [1974]; zu Nebenreaktionen Base und Acrylnitril in Gegenwart von Pyrrol, vergleiche z. B. Liebigs Ann. Chem. 615, 124 [1958]; J. Chem. Soc, 1962, 4346.)

Weiterhin ist es überraschend, daß die Umsetzung der Bromide der Formel (II) mit Acrylnitril in Gegenwart von Palladium in der ersten Stufe des erfindungugemäßen Verfahrens ohne den in der Literatur beschriebenen Zusatz von Phosphinen als Reaktionshilfsmittel sehr hohe Ausbeuten erbringt, wobei zudem die in der Literatur beschriebenen Reaktionsbedingungen (vergleiche z. B. EP 78 768 oder US 4.820.835 oder J. Chem. Soc., Perkin Trans 1, 1987, 2597 oder J. Organomet. Chem. 258, 101, [1983]) im Falle der mindestens im Arylteil 2,3-disubstituierten Arylverbindungen, welche als Fungizide von besonderem Interesse sind, versagen; es findet unter den in der Literatur angegebenen Bedingungen fast keine Reaktion statt.

Das erfindungsgemäße Verfahren besitzt gegenüber den vorbekannten Verfahren eine Reihe von Vorteilen:

So hat man beispielsweise durch die Vermeidung von Phosphinen als Reaktionshilfsmittel in der ersten Stufe erheblich weniger Abwasserprobleme, da sich Phosphine und ihre Reaktionsprodukte - insbesondere auch Phosphinoxide - durch ihre hohe Wasserlöslichkeit nur sehr schwer aus dem anfallenden Abwasser entfernen lassen.

Ein weiterer Vorteil ist die Vermeidung von technisch schwierig zu handhabendem und aufwendige Sicherheitsvorkehrungen erforderndem Natriumhydrid in der zweiten Stufe, Die bei dem erfindungsgemäßen Verfahren als Basen verwendeten Alkalimetallalkoholate oder Alkalimetallhydroxide sind billig herzustellen, einfach zu handhaben und sicherheitstechnisch unbedenklich.

Aus dem Stand der Technik war weiterhin bekannt, daß man das Phenylsulfonylmethylisocyanid ersetzen kann durch substituierte Phenylsulfonylmethylisocyanide wie 4-Chlorphenyl- oder 4-Methylphenylsulfonylmethylisocyanid. Es war aber bisher nicht bekannt, daß die substituierten Verbindungen zusätzliche Vorteile erbringen. So zeigt die 4-Chlorphenyl-Verbindung eine geringe Thermolabilität, was den sicherheitstechnischen Spielraum erweitert und ist außerdem im Hinblick auf Herstellung und Rückgewinnung aus seinen Reaktionsendprodukten (Natrium- oder Kalium-p-Chlorphenylsulfinat) wirtschaftlicher einsetzbar. Auch die 4-Methylphenyl-Verbindung ist hier einsetzbar, da die 2. Stufe des erfindungsgemäßen Verfahrens schon bei niedrigen Reaktionstemperaturen sehr hohe Ausbeuten erbringt und man somit nicht in den Temperaturbereich vordringt, in dem die thermische Labilität des p-Toluolsulfonylmethylisocyanids anfängt problematisch zu werden.

Der letzte aber nicht unwesentlichste Vorteil des erfindungsgemäßen Verfahrens liegt in einer beträchtlichen Steigerung der Gesamtausbeute an fungizidem Endprodukt im Vergleich zu den vorbekannten Verfahren.

Verwendet man beispieleweise 1-Brom-2-fluor-3-chlorphenyl als Ausgangsverbindung, so läßt sich der Reaktionsablauf des erfindungsgemäßen Verfahrens durch das folgende Formelschema darstellen:

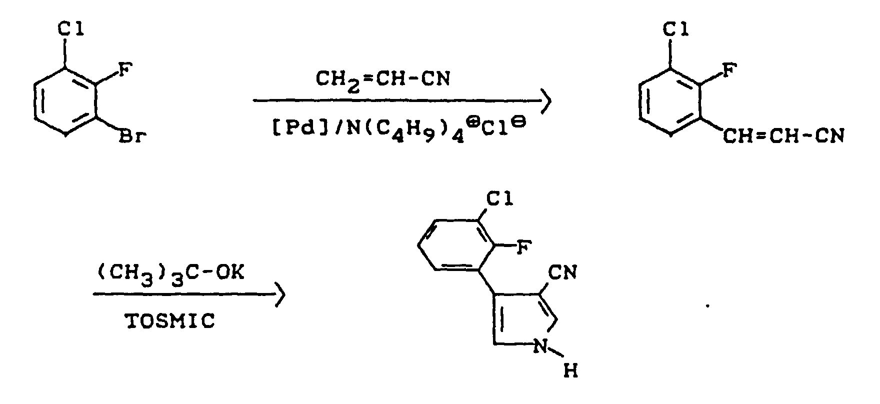

Die zur Durchführung des erfindungegemäßen Verfahrens als Ausgangsstoffe benötigten Bromide der Formel (II) sind nur teilweise bekannt (vergleiche z. B. J. Chem. Soc. Perkin Trans. 2, 1972, 1733 oder J. Chem. Soc. 79, 1302 [1901] J. Amer. Chem. Soc. 87, 2640 [1965] oder Org. Magn. Reson. 9, 155 [1977] oder Circ. III. State Geol. Surv. 1979, 508 (17 pp.) bzw. CA 92:53358d).

Neu sind Verbindungen der Formel (IIa),

Ar¹-Br (IIa)

in welcher
- Ar¹: für 2-Fluor-3-chlorphenyl, für 2-Fluor-3-trifluormethylphenyl oder für 2,2- Difluorbenzodioxol-4-yl steht.

Verbindungen der Formel (IIa) sind ebenfalls Gegenstand der Erfindung.

Man erhält die neuen und bekannten Bromide der Formeln (II) bzw. (IIa), indem man Amine der Formel (V)

Ar-NH₂ (V)

bzw.

Ar¹-NH₂ (Va)

in welcher
- Ar und Ar¹: die angegebene Bedeutung haben,
mit Natriumnitrit in Gegenwart von Bromwasserstoff und eines Katalysators und in Gegenwart eines Lösungsmittels umsetzt.

Alternativ ist es auch möglich, die Verbindung der Formel (IIb), 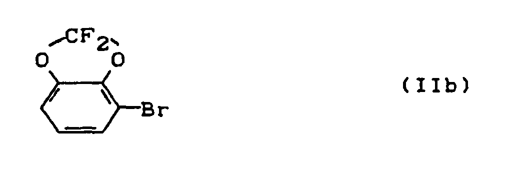 herzustellen, indem man 2,2-Difluorbenzodioxol (vergleiche z. B. DE 19 21 741) zunächst mit n-Butyllithium in Gegenwart eines Verdünnungsmittels wie beispielsweise Tetrahydrofuran bei Temperaturen zwischen -80°C und -60°C umsetzt, anschließend elementares Brom zugibt, in üblicher Art und Weise mit verdünnter Salzsäure bei Raumtemperatur hydrolysiert und nach bekannten Verfahren aufarbeitet (vergleiche hierzu beispielsweise EP 333 658 oder die Herstellungsbeispiele).

Phenylsulfonylmethylisocyanide der Formel (IV) sind bekannt oder erhältlich mit Hilfe bekannter Verfahren (vergleiche z. B. DE-OS 36 01 285 oder US 4.680.413 oder Tetrahedron Lett. 1972, 2367 oder J. Org. Chem. 42, 1153 [1977] oder Synthesis 1985, 400 oder Organic Syntheses 57, 102 [1977]).

Als Verdünnungsmittel zur Herstellung der Ausgangsbromide der Formel (II) kommen alle dir solche Diazotierungsreaktionen üblichen Verdünnungumittel infrage. Bevorzugt verwendet man wässrige Systeme, insbesondere wässrige Protonensäuren, wie beispielsweise Schwefelsäure, Salpetersäure, Salzsäure oder Bromwasserstoffsäure.

Das Herstellungsverfahren der Bromide (II) wird in Gegenwart eines geeigneten Katalysators durchgeführt. Als solche kommen mit besonderem Vorzug Kupfer-(I)-Salze, wie beispielsweise Kupfer-(I)-chlorid, Kupfer(I)-iodid sowie insbesondere Kupfer-(I)-bromid infrage.

Die Reaktionstemperaturen können in einem größeren Bereich variiert werden. Im allgemeinen arbeitet man bei Temperaturen zwischen -20°C und +120°C, vorzugsweise bei Temperaturen zwischen +5°C und +90°C.

Zur Durchführung des Verfahrens zur Herstellung des Ausgangsproduktes setzt man pro Mol an Amin der Formel (V) im allgemeinen 1,0 bis 5,0 Mol, vorzugsweise 1,0 bis 2,5 Mol an Natriumnitrit, 1,0 bis 10,0 Mol, vorzugsweise 1,0 bis 5,0 Mol an hochkonzentrierter Bromwasserstoffsäure und 0,01 bis 1,0 Mol, vorzugsweise 0,05 bis 0,5 Mol an Kupfer-(I)-Katalysator ein. Die Reaktionsdurchführung erfolgt in Analogie zu bekannten Standardverfahren (vergleiche z. B. Houben-Weyl "Methoden der organischen Chemie" Band V,1 S.441 oder Organic Syntheses Coll. Vol. III, 185 [1955]). Um Nebenreaktionen zu vermeiden sollte die Reaktionstemperatur 40°C nur für kurze Zeit übersteigen. Die Aufarbeitung und Isolierung der Reaktionsprodukte erfolgt nach üblichen, allgemein bekannten Methoden (vergleiche auch die Herstellungsbeispiele).

Die erste Stufe des erfindungsgemäßen Verfahrens wird in Gegenwart eines geeigneten Palladiumkatalysators durchgeführt. Als solche kommen Palladiumsalze, wie beispielsweise Palladiumchlorid, Palladiumacetat oder Palladiumsulfat oder elementares Palladium gegebenenfalls auf einem geeigneten Träger wie beispielsweise Aktivkohle oder Siliciumdioxid infrage.

Als Verdünnungsmittel zur Durchführung der ersten Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Tolual, Xylol, Chlorbenzol, Dichlorbenzol, Petrolether, Hexan, Cyclohexan, Tetrachlorkohlenstoff; Ether, wie Diethylether, Diisopropylether, Dioxan, Tetrahydrofuran, Ethylenglykoldimethyl- oder -diethylether oder Diethylenglykoldimethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester oder Essigsäureethylester; Sulfoxide, wie Dimethylsulfoxid oder Alkohole, wie Methanol, Ethanol, n- oder i-Propanol, n-Butanol, i- Butanol, s-Butanol, Ethylenglykolmonomethylether oder Ethylenglykolmonoethylether.

Aprotisch polare Lösungsmittel, wie Tetrahydrofuran, Dioxan, Ethylenglykol-dimethylether und -diethylether, Diethylenglykol-dimethylether, Dimethylformamid, Dimethylacetamid und N-Methyl-pyrrolidon sind als Verdünnungsmittel zur Durchführung der ersten Stufe des erfindungsgemäßen Verfahrens besonders bevorzugt.

Die erste Stufe des erfindungsgemäßen Verfahrens wird in Gegenwart eines geeigneten Reaktionshilfmittels durchgeführt. Als solche kommen alle üblichen anorganischen oder organischen Basen infrage. Hierzu gehören beispielsweise Erdalkali- oder Alkalimetallhydroxide, wie Natriumhydroxid, Calciumhydroxid, Kaliumhydroxid oder auch Ammoniumhydroxid, Alkylimetallcarbonate, wie Natriumcarbonat, kaliumcarbonat, Kaliumhydrogencarbonat, Natriumhydrogencarbonat oder Ammoniumcarbonat, Alkali- oder Erdalkalimetallacetate, wie Natriumacetat, Kaliumacetat, Calciumacetat oder Ammoniumacetat, sowie tertiäre Amine, wie Trimethylamin, Triethylamin, Tributylamin, N,N-Dimethylanilin, Pyridin, Piperidin, N-Methylpiperidin, N,N-Dimethylaminopyridin, Diazabicylooctan (DABCO), Diazabicyclononen (DBN) oder Diazabicycloundecen (DBU).

Außerdem ist es vorteilhaft, als weiteres Reaktionshilfsmittel einen geeigneten Phasentransferkatalysator zuzusetzen. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethylammoniummethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der ersten Stufe des erfindungsgemäßen Verfahrens in einem größeren Bereich variiert werden. Im allgemeinen arbeitet man bei Temperaturen zwischen 50°C und 250°C, vorzugsweise bei Temperaturen zwischen 100°C und 150°C.

Das erfindungsgemäße Verfahren wird im allgemeinen bei Normaldruck durchgeführt. Es ist jedoch auch möglich unter vermindertem oder erhöhtem Druck zu arbeiten. Möglich sind Druckbereiche zwischen 0,5 und 100 bar, vorzugsweise zwischen 0,9 und 10 bar.

Zur Durchführung der ersten Stufe des erfindungsgemäßen Verfahrens setzt man pro Mol an Bromid der Formel (II) im allgemeinen 1,0 bis 5,0 Mol, vorzugsweise 1,0 bis 2,0 Mol an Acrylnitril, 0,1 bis 0,0001 Mol, vorzugsweise 0,01 bis 0,001 Mol an Palladiumkatalysator, gegebenenfalls 1,0 bis 5,0 Mol, vorzugsweise 1,0 bis 2,5 Mol an als Reaktionshilfsmittel verwendeter Base und gegebenenfalls 0,01 bis 2,0 Mol, vorzugsweise 0,1 bis 1,5 Mol an Phasentransferkatalysator ein. Die Reaktionsdurchführung, Aufarbeitung und Isolierung der Reaktionsprodukte erfolgt nach bekannten Verfahren (vergleiche hierzu die Herstellungsbeispiele).

Als Verdünnungemittel zur Durchführung der zweiten Stufe des erfindungsgemäßen Verfahrens kommen inerte organische Lösungsmittel infrage. Hierzu gehören insbesondere aliphatische, alicyclische oder aromatische, gegebenenfalls halogenierte Kohlenwasserstoffe, wie beispieleweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Methyl-tert-butylether, Dioxan, Tetrahydrofuran oder Ethylenglykoldimethyl- oder di-ethylether; Nitrile, wie Acetonitril, Propionitril oder Benzonitril; Amide, wie N,N-Dimethylformamid, N,N-Dimethylacetamid, N-Methylformanilid, N-Methylpyrrolidon oder Hexamethylphosphorsäuretriamid; Ester, wie Essigsäuremethylester, Essigsäureethylester; Alkohole, wie Methanol, Ethanol, n- oder i-Propanol, Ethylenglykolmonomethylether oder Ethylenglykolmonoethylether.

Die zweite Stufe des erfindunggemäßen Verfahren wird in Gegenwart eines Alkalimetallalkoholates oder Alkalimetallhydroxids, wie beispielsweise Natriummethylat, Natriumethylat, Natriumisobutylat, Kalium-tertbutylat, Natriumhydroxid oder Kaiiumhydroxid, durchgeführt.

Bei Verwendung eines unpolaren Lösungsmittels ist es außerdem von Vorteil, als weiteres Reaktionshilfsmittel einen geeigneten Phasentransferkatalysator zuzusetzen. Als Beispiele für solche Katalysatoren seien genannt: Tetrabutylammoniumiodid, Tetrabutylammoniumbromid, Tetrabutylammoniumchlorid, Tributyl-methylphosphoniumbromid, Trimethyl-C₁₃/C₁₅-alkylammoniumchlorid, Trimethyl-C₁₃/C₁₅-alkylammoniumbromid, Dibenzyl-dimethyl-ammoniummnethylsulfat, Dimethyl-C₁₂/C₁₄-alkyl-benzylammoniumchlorid, Dimethyl-C₁₂/C₁₄-alkyl-benzylammoniumbromid, Tetrabutylammoniumhydroxid, Triethylbenzylammoniumchlorid, Methyltrioctylammoniumchlorid, Trimethylbenzylammoniumchlorid, 15-Krone-5, 18-Krone-6 oder Tris-[2-(2-methoxyethoxy)-ethyl]-amin.

Die Reaktionstemperaturen können bei der zweiten Stufe des erfindungsgemäßen Verfahrens in einem größeren Bereich variiert werden. Im allgemeinen arbeitet man bei Temperaturen zwischen -30°C und +100°C, vorzugsweise bei Temperaturen zwischen -30°C und +40°C.

Zur Durchführung der zweiten Stufe des erfindungsgemäßen Verfahrens setzt man pro Mol an Acrylnitril-Derivat der Formel (III) im allgemeinen 1,0 bis 2,0 Mol, vorzugsweise 1,0 bis 1,3 Mol an Phenylsulfonylmethylisocyanid der Formel (IV), 1,2 bis 1,8 Mol an Base und gegebenenfalls 0,01 bis 2,0 Mol, vorzugsweise 0,1 bis 1,5 Mol an Phasentransferkatalysator ein.

Es kommt dabei bei Verwendung von schlecht solvatisierenden Lösungsmitteln, wie z. B. Toluol oder Methyltert-butylether auf die Zugabe eines der oben genannten Phasentransferkatalysatoren und/oder auf die Beimischung eines polareren Lösungsmittels wie beispielsweise Tetrahydrofuran an, wodurch es gelingt, die Reaktivität der verwendeten Base und des Phenylsulfonylmethylisocyanids der Formel (IV) so abzustufen, daß eine glatte Umsetzung erfolgt, ohne daß die Reaktionstemperatur in den Bereich erhöht werden muß, wo die Gefahr exothermer Zersetzung des Phenylsulfonylmethylisocyanids der Formel (IV) besteht.

Man geht dabei so vor, daß zunächst das Acrylnitril-Derivat der Formel (III) zusammen mit der Base und gegebenenfalls dem Phasentransferkatalysator in einem geeigneten Lösungsmittel vorgelegt wird, anschließend das Phenylsulfonylmethylisocyanid der Formel (IV), in einem geeigneten Lösungsmittel gelöst, tropfenweise dazugegeben wird, danach für einige Stunden bei der erforderlichen Temperatur (in der Regel Raumtemperatur) gerührt wird, ausgefallenes Phenylsulfinat abfiltriert wird, organisches Lösungsmittel im Vakuum entfernt wird und der Rückstand mit Wasser behandelt wird, wobei die gewünschten in 3-Stellung substituierten 4-Cyanopyrrolverbindungen der Formel (I) als Niederschlag anfallen und alle Nebenprodukte in Lösung bleiben. Durch Filtration und Trocknung erhält man Produkte von hoher Reinheit.

In einer Variante des erfindungsgemäßen Verfahrens ist es auch möglich, die erste und die zweite Stufe in einem Reaktionsschritt ohne Isolierung der Acrylnitril-Derivate der Formel (III) in einem sogenannten "Eintopfverfahren" durchzuführen (vergleiche hierzu auch die Herstellungsbeispiele).

Die mit Hilfe des erfindungsgemäßen Verfahrens erhältlichen in 3-Stellung substituierten 4-Cyano-pyrrolverbindungen sind bekannte Fungizide für den landwirtschaftlichen Gebrauch (vergleiche z. B. EP 293 711 oder EP 315 869 oder DE-OS 37 37 984 oder DE-OS 38 00 387).

### Herstellungsbeispiele

### Beispiel 1:

### Variante a)

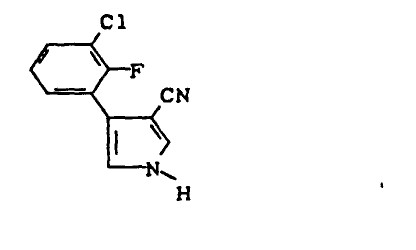

8,65 g (0,077 Mol) t-Kaliumbutylat werden in 100 ml Tetrahydrofuran vorgelegt und bei -10°C mit einer Mischung aus 10 g (0,055 Mol) 2-Fluor-3-chlorzimtsäurenitril und 14 g (0,071 Mol) p-Toluolsulfonylmethylisocyanid (TOSMIC) in 200 ml Tetrahydrofuran versetzt. Nach 3stündigem Rühren bei Raumtemperatur wird das Tetrahydrofuran im Vakuum abdestilliert, der Rückstand in Wasser aufgenommen und der ausgefallene Niederschlag abgesaugt und getrocknet.

Man erhält 12,0 g (95 % der Theorie) an 3-Cyano-4-(2-fluor-3-chlorphenyl)-pyrrol (Gehalt: 95 %; Bestimmung durch HPLC), vom Schmelzpunkt 180°C bis 181°C.

### Beispiel 1:

### Variante b)

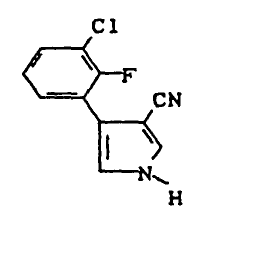

1,3 g (0,023 Mol) Kaliumhydroxyd-Pulver werden in 30 ml Tetrahydrofuran vorgelegt, mit 9 ml Tris-[2-(2-methoxyethoxy)ethyl]-amin versetzt und bei -10°C mit einer Mischung aus 3 g (0,016 Mol) 2-Fluor-3-chlorzimtsäurenitril und 4,2 g (0,022 Mol) p-Toluolsulfonylmethylisocyanid (TOSMIC) in 60 ml Tetrahydrofuran versetzt. Nach 3stündigem Rühren bei Raumtemperatur wird das Tetrahydrofuran im Vakuum abdestilliert, der Rückstand in Wasser aufgenommen und der ausgefallene Niederschlag abgesaugt und getrocknet.

Man erhält 2,9 g (73 % der Theorie) 3-Cyano-4-(2-fluor-3-chlorphenyl)-pyrrol (Gehalt 91 %; Bestimmung durch Gaschromatographie) vom Schmelzpunkt 180°C.

### Beispiel 1:

### Variante c)

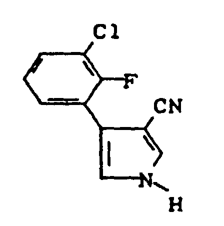

Zu 1,65 g (0,009 Mol) 2-Fluor-3-chlorzimtsäurenitril und 2,4 g (0,01 Mol) p-Chlorphenylsulfonylmethylisocyanid in 20 ml Dimethoxyethan gibt man bei 0°C unter Rühren 1,35 g (0,015 Mol) Natriumhydroxid gelöst in wenig Wasser und rührt nach beendeter Zugabe eine weitere Stunde bei 0°C. Zur Aufarbeitung wird das Lösungsmittel im Vakuum abdestilliert, der Rückstand in Wasser aufgenommen, der ausgefallene Niederschlag abgesaugt und getrocknet.

Man erhält 1,65 g (81 % der Theorie) an 3-Cyano-4-(2-fluor-3-chlorphenyl)-pyrrol (Gehalt 98 %; Bestimmung durch Gaschromatographie) vom Schmelzpunkt 180°C bis 181°C.

### Analog können Beispiel 2

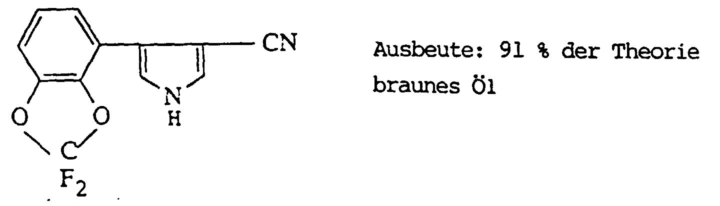

### und

### Beispiel 3 hergestellt werden

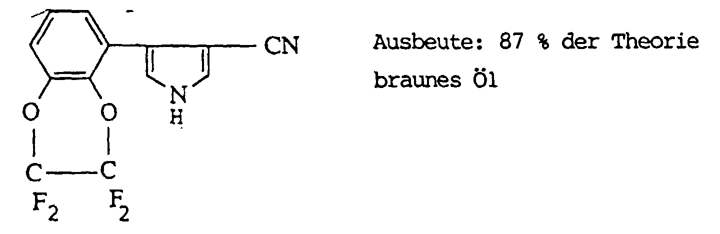

### Beispiel 1:

### Variante d)

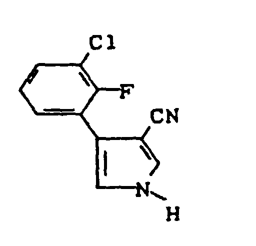

### (Eintopfverfahren)

3,0 g (0,014 Mol) 1-Brom-2-fluor-3-chlorbenzol und 1,15 g (0,021 Mol) Acrylnitril werden in 100 ml Dimethylformamid vorgelegt, mit 0,07 g (0,3 mMol) Palladiumacetat, 3 g (0,036 Mol) Natriumhydrogencarbonat und 1,7 g (0,006 Mol) Tetrabutylammoniumchlorid versetzt und unter einer Stickstoffatmosphäre auf 120°C erhitzt. Nach 16stündiger Reaktionszeit wird das Reaktionsgemisch abgesaugt, das Filtrat zusammen mit 3,65 g (0,019 Mol) p-Toluolsulfonylmethylisocyanid (TOSMIC) bei -10°C zu 2,25 g (0,020 Mol) t-Kaliumbutylat in 20 ml Dimethylformamid getropft. Nach 3stündigem Rühren bei Raumtemperatur wird die Reaktionsmischung in Wasser gegeben und der ausgefallene Niederschlag abgesaugt und getrocknet.

Man erhalt 2,65 g (67 % der Theorie) an 3-Cyano-4-(2-fluor-3-chlorphenyl)-pyrrol (Gehalt 90 %; Bestimmung durch Gaschromatographie) vom Schmelzpunkt 178°C.

### Vorproduktherstellung

### Beispiel III-1

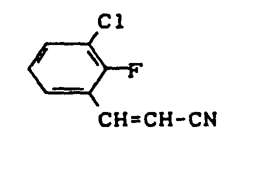

5,0 g (0,024 Mol) 1-Brom-2-fluor-3-chlorbenzol und 1,9 g (0,036 Mol) Acrylnitril werden in 150 ml Dimethylformamid vorgelegt und mit 0,11 g (0,5 mMol) Palladiumacetat, 5 g (0,060 Mol) Natriumhydrogencarbonat und 2,85 g (0,010 Mol) Tetrabutylammoniumchlorid versetzt und auf 120°C erhitzt. Nach 16stündiger Reaktionszeit wird das Reaktionsgemisch in Wasser gegossen, mit Essigester extrahiert mit Wasser und 2 normaler Salzsäure gewaschen, getrocknet und im Vakuum vom Lösungsmittel befreit.

Man erhält 4,1 g (89 % der Theorie) an 2-Fluor-3-chlorzimtsäurenitril (Gehalt 99 %; Bestimmung durch Gaschromatographie) vom Schmelzpunkt 92°C bis 94°C.

### Analog kann Beispiel III-2 hergestellt werden:

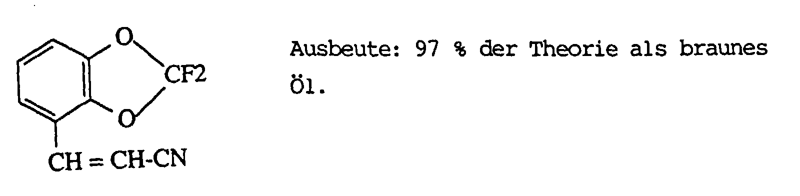

### Beispiel III-1 (Vergleichsbeispiel):

### (vergleiche hierzu J. Chem. Soc., Perkin Trans 1, 1987, 2597 sowie J. Organometall. Chem. 258, 101, [1983])

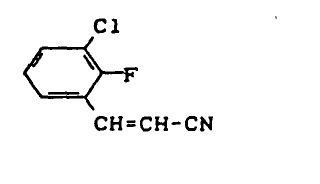

Zu 0,3 g (0,0014 Mol) 1-Brom-2-fluor-3-chlor-benzol und 0,1 g (0,0018 Mol) Acrylnitril in 10 ml Triethylamin werden 0,0032 g (0,015 mMol) Palladiumacetat und 0,015 g (0,058 mMol) Triphenylphosphin gegeben. Nach 7stündiger Reaktion bei Rückflußtemperatur wird das Reaktionsgemisch gaschromatographisch analysiert. Das gewünschte Produkt (2-Fluor-3-chlorzimtsäurenitril) wird dabei nicht gefunden.

### Beispiel II-1

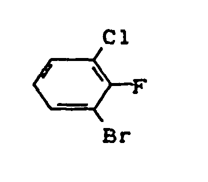

Zu 80 ml einer 65 %igen Schwefelsäure gibt man bei Raumtemperatur tropfenweise unter Rühren 14,6 g (0,1 Mol) 2-Fluor-3-chloranilin, kühlt dann auf 0°C ab, gibt tropfenweise eine Lösung aus 11 g (0,16 Mol) Natriumnitrit in 30 ml Wasser dazu, rührt bei 5°C 30 Minuten nach, und gibt dann diese Lösung portionsweise zu einem Gemisch aus 45 ml 48 %iger Bromwasserstoffsäure und 2,8 g Kupfer(I)bromid. Man rührt die Reaktionsmischung eine Stunde bei Raumtemperatur und anschließend 90 Minuten bei 90°C. Zur Aufarbeitung wird die Reaktionslösung abgekühlt, mit 200 ml Wasser verdünnt, dreimal mit insgesamt 300 ml Methyl-tert-butylether extrahiert, mit Wasser gewaschen, über Natriumsulfat getrocknet und im Vakuum vom Lösungsmittel befreit.

Man erhält 21 g (97 % der Theorie) an 1-Brom-3-chlor-2-fluorbenzol vom Schmelzpunkt 43°C und vom Siedepunkt 88°C bis 92°C bei 32 mbar.

### Beispiel II-2:

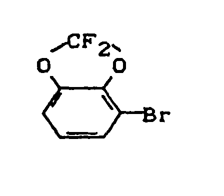

Zu 15,8 g (0,1 Mol) 2,2-Difluorbenzodioxol (vergleiche z. B. DE-OS 19 21 741) in 80 ml absolutem Tetrahydrofuran gibt man unter einer trockenen Stickstoffatmosphäre bei -78°C innerhalb von 1 Stunde unter Rühren tropfenweise 45 ml (0,11 Mol) n-Butyllithium (2,5 molar in n-Hexan), rührt anschließend 1 Stunde bei -78°C und gibt dann ebenfalls tropfenweise unter Rühren bei -78°C 16 g (0,1 Mol) Brom zu. Man rührt weitere 90 Minuten bei -78°C, läßt dann die Mischung auf Raumtemperatur kommen und hydrolysiert mit 75 ml 10prozentiger Salzsäure. Zur Aufarbeitung trennt man die organische Phase ab, wäscht mit 1 normaler Salzsäure, trocknet über Natriumsulfat, engt im Vakuum ein und destilliert den Rückstand im Wasserstrahlvakuum.

Man erhält 11,3 g (46 % der Theorie) an 4-Brom-2,2-difluorbenzodioxol vom Siedepunkt 74°C bis 75°C bei 20 mbar.

## Patentansprüche

1. Verfahren zur Herstellung von in 3-Stellung substituierten 4-Cyanopyrrolverbindungen der Formel 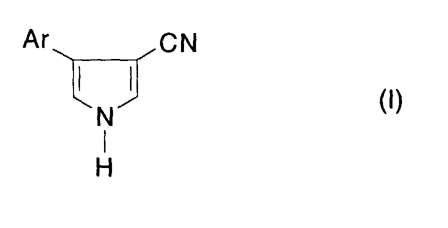 in welcher
Ar für 2,3-Dichlorphenyl, 2,3-Difluorphenyl, 2-Fluor-3-chlorphenyl, 2-Chlor-3-fluorphenyl, 2,4-Difluor-3-chlorphenyl, 2-Fluor-3-trifluormethylphenyl oder 2,2-Difluorbenzodioxolyl steht,
durch Umsetzung von Bromiden der Formel
Ar―Br (II)
in welcher
Ar die oben angegebene Bedeutung hat,
in erster Stufe mit Acrylnitril in Gegenwart eines Lösungsmittels und eines geeigneten Reaktionshilfsmittels zu den Acrylnitril-Derviaten der Formel
Ar―CH=CH―CN (III)
in welcher
Ar die oben angegebene Bedeutung hat,
und
in einer zweiten Stufe mit Phenylsulfonylmethylisocyaniden der Formel
Ph―SO₂―CH₂―NC (IV)
in welcher
Ph für 4-Methylphenyl oder 4-Chlorphenyl steht,
in Gegenwart eines geeigneten Verdünnungsmittels und gegebenenfalls in Gegenwart eines geeigneten Reaktionshilfsmittels, dadurch gekennzeichnet, daß man die erste Stufe in Gegenwart eines Palladiumkatalysators und die zweite Stufe in Gegenwart eines 1,2- bis 1,8fach molaren Überschusses an Alkalimetallalkoholat oder Alkalimetallhydroxid, bezogen auf Acrylnitril-Derivat, durchführt.

2. Verfahren nach Anspruch 1, dadurch gekennzeichnet, daß als Palladiumkatalysatoren in der ersten Stufe Palladiumsalze oder elementares Palladium auf einem geeigneten Träger verwendet werden.

3. Verfahren nach Anspruch 1, dadurch gekennzeichnet, daß die in erster Stufe erhaltenen Acrylnitril-Derivate der Formel (III) zwischenzeitlich nicht isoliert, sondern direkt in der Reaktionslösung weiter verarbeitet werden.

4. Verfahren nach Anspruch 1, dadurch gekennzeichnet, daß man in der ersten Stufe im Temperaturbereich von 50 °C bis 250 °C und in der zweiten Stufe von - 30 °C bis + 100 °C arbeitet.

5. Bromide der Formel
Ar¹―Br (IIa)
in welcher
Ar¹ für 2-Fluor-3-chlorphenyl, 2-Fluor-3-trifluormethylphenyl oder 2,2-Difluorbenzodioxol-4-yl steht.

6. Verfahren zur Herstellung von Bromiden der Formel (IIa) gemäß Anspruch 5,
dadurch gekennzeichnet daß man Amine der Formel
Ar¹―NH₂ (Va)
in welcher
Ar¹ die oben angegebene Bedeutung hat,
mit Natriumnitrit in Gegenwart von Bromwasserstoff und eines Katalysators und in Gegenwart eines Lösungsmittels umsetzt.

## Claims

1. Process for the preparation of 3-substituted 4-cyano-pyrrole compounds of the formula (I), 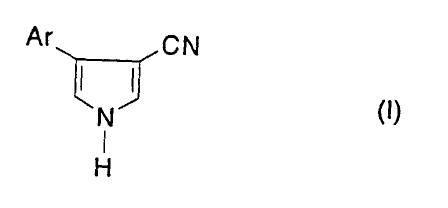 in which
Ar represents 2,3-dichlorophenyl, 2,3-difluorophenyl, 2-fluoro-3-chlorophenyl, 2-chloro-3-fluorophenyl, 2,4-difluoro-3-chlorophenyl, 2-fluoro-3-trifluoromethylphenyl or 2,2-difluorobenzodioxolyl,
by reaction of bromides of the formula
Ar―Br (II)
in which
Ar has the meaning given above,
in a first stage with acrylonitrile in the presence of a solvent and a suitable reaction auxiliary, to give the acrylonitrile derivatives of the formula
Ar―CH=CH―CN (III)
in which
Ar has the meaning given above
and
in a second stage with phenylsulphonylmethyl isocyanides of the formula
Ph―SO₂―CH₂―NC (IV)
in which
Ph represents 4-methylphenyl or 4-chlorophenyl,
in the presence of a suitable diluent and optionally in the presence of a suitable reaction auxiliary, characterised in that the first stage is carried out in the presence of a palladium catalyst and the second stage in the presence of a 1.2- to 1.8-fold molar excess of alkali metal alcoholate or alkali metal hydroxide, based on acrylonitrile derivative.

2. Process according to Claim 1, characterised in that the palladium catalysts used in the first stage are palladium salts or elemental palladium on a suitable support.

3. Process according to Claim 1, characterised in that the acrylonitrile derivatives of the formula (III) obtained in the first stage are not isolated intermediately, but are further processed directly in the reaction solution.

4. Process according to Claim 1, characterised in that in the first stage a temperature range from 50°C to 250°C, and in the second stage from -30°C to +100°C is used.

5. Bromides of the formula
Ar¹―Br (IIa)
in which
Ar¹ represents 2-fluoro-3-chlorophenyl, 2-fluoro-3-trifluoromethylphenyl or 2,2-difluorobenzodioxol-4-yl.

6. Process for preparation of bromides of the formula (IIa) according to Claim 5,
characterised in that amines of the formula
Ar¹―NH₂ (Va)
in which
Ar¹ has the meaning given above,
are reacted with sodium nitrite in the presence of hydrogen bromide and a catalyst and in the presence of a solvent.

## Revendications

1. Procédé pour la préparation de composés de 4-cyanopyrrole substitués en position 3, répondant à la formule 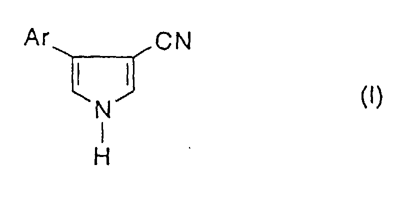 dans laquelle
Ar représente un groupe 2,3-dichlorophényle, un groupe 2,3-difluorophényle, un groupe 2-fluoro-3-chlorophényle, un groupe 2-chloro-3-fluorophényle, un groupe 2,4-difluoro-3-chlorophényle, un groupe 2-fluoro-3-trifluorométhylphényle ou un groupe 2,2-difluorobenzodioxolyle,
par mise en réaction de bromures répondant à la formule
Ar―Br (II)
dans laquelle
Ar a la signification indiquée ci-dessus,
dans une première étape avec de l'acrylonitrile en présence d'un solvant et d'un adjuvant réactionnel approprié pour obtenir les dérivés d'acrylonitrile répondant à la formule
Ar―CH=CH―CN (III)
dans laquelle
Ar a la signification indiquée ci-dessus
et
dans une deuxième étape avec des phénylsulfonylméthylisocyanures répondant à la formule
Ph―SO₂―CH₂―NC (IV)
dans laquelle
Ph représente un groupe 4-méthylphényle ou un groupe 4-chlorophényle,
en présence d'un diluant approprié et le cas échéant en présence d'un adjuvant réactionnel approprié, caractérisé en ce qu'on effectue la première étape en présence de palladium à titre de catalyseur et la seconde étape en présence d'un excès de 1,2 à 1,8 fois molaire d'alcoolate de métal alcalin ou d'hydroxyde de métal alcalin rapporté au dérivé d'acrylonitrile.

2. Procédé selon la revendication 1, caractérisé en ce qu'on utilise, à titre de catalyseurs de palladium dans la première étape, des sels de palladium ou du palladium élémentaire sur un support approprié.

3. Procédé selon la revendication 1, caractérisé en ce qu'on ne soumet pas à une isolation intermédiaire les dérivés d'acrylonitrile répondant à la formule (III) obtenus à la première étape, mais on les soumet directement à un traitement ultérieur dans la solution réactionnelle.

4. Procédé selon la revendication 1, caractérisé en ce qu'on travaille dans la première étape dans le domaine de température de 50°C à 250°C et dans la seconde étape de -30°C à +100°C.

5. Bromures répondant à la formule
Ar¹―Br (IIa)
dans laquelle
Ar¹ représente un groupe 2-fluoro-3-chlorophényle, un groupe 2-fluoro-3-trifluorométhylphényle ou un groupe 2,2-difluorobenzodioxol-4-yle.

6. Procédé pour la préparation de bromures répondant à la formule (IIa) selon la revendication 5,
caractérisé en ce qu'on fait réagir des amines répondant à la formule
Ar¹―NH₂ (Va)
dans laquelle
Ar¹ a la signification indiquée ci-dessus,
avec du nitrite de sodium en présence d'acide bromhydrique et d'un catalyseur et en présence d'un solvant.
